# EUROPEAN PATENT SPECIFICATION

(11) **EP 1 756 289 B1**
(45) Date of publication and mention of the grant of the patent: **14.01.2015**
(21) Application number: 05746540.3
(22) Date of filing: 24.05.2005
(51) Int. Cl.: A61K 47/48, C12N 15/113, G01N 33/58, A61K 49/00, A61K 51/12, A61K 49/18, C12N 15/88, B82Y 5/00, B82Y 15/00

(54) **NANOPARTICLES COMPRISING RNA LIGANDS**
RNA-LIGANDEN UMFASSENDE NANOPARTIKEL
NANOPARTICULES COMPRENANT DES LIGANDS D'ARN

(30) Priority: 24.05.2004 US 573805 P; 24.05.2004 GB 0411537
(43) Date of publication of application: 28.02.2007
(60) Divisional application: 10181903.5
(73) Proprietor: Midatech Ltd., Abingdon, Oxford Oxfordshire 0X13 6BH (GB)
(72) Inventor: RADEMACHER, Thomas William, Midatech Ltd, Abingdon, Oxford Oxfordshire OX13 6BH (GB); GUMAA, Khalid, Midatech Ltd, Abingdon, Oxford Oxfordshire OX13 6BH (GB); MARTIN-LOMAS, Manuel, 28006 Madrid (ES); PENADES, Soledad, 28006 Madrid (ES); OJEDA, Rafael, 28006 Madrid (ES); BARRIENTES, Africa G., 28006 Madrid (ES)
(74) Representative: Kiddle, Simon John
(86) International application number: PCT/GB2005/002058
(87) International publication number: WO 2005/116226

(56) References cited:
- EP-A- 1 249 502
- EP-A1- 0 990 903
- WO-A-02/32404
- WO-A-98/04740
- WO-A1-01/00876
- WO-A2-01/51665
- US-A1- 2003 147 966
- PATOLSKY F ET AL: "Dendritic amplification of DNA analysis by oligonucleotide-functionalized Au-nanoparticles" CHEMICAL COMMUNICATIONS - CHEMCOM, vol. 6, no. 12, 21 June 2000 (2000-06-21), pages 1025-1026, XP002229552 ISSN: 1359-7345
- MATZKE M ET AL: "RNAi extends its reach" SCIENCE, vol. 301, no. 5636, 22 August 2003 (2003-08-22), pages 1060-1061, XP002432809 ISSN: 0036-8075
- MIYAGISHI M ET AL: "Comparison of the suppressive effects of antisense oligonucleotides and siRNAs directed against the same targets in mammalian cells" ANTISENSE AND NUCLEIC ACID DRUG DEVELOPMENT, vol. 13, no. 1, 2003, pages 1-7, XP002432810 ISSN: 1087-2906
- CHOUDHURY A ET AL: "Small interfering RNA (siRNA) inhibits the expression of the Her2/Neu gene, upregulates HLA class I and induces apoptosis of Her2/Neu positive tumor cell lines" INTERNATIONAL JOURNAL OF CANCER, vol. 108, no. 1, 1 January 2004 (2004-01-01), pages 71-77, XP002432811 ISSN: 0020-7136

## Description

### Field of the Invention

The present invention relates to nanoparticles, and more particularly to nanoparticles comprising RNA ligands such as small interfering RNA (siRNA) and micro RNA (miRNA), and their use in a range of applications.

### Background of the Invention

Small RNA molecules have been found to play multiple roles in regulating gene expression. These include targeted degradation of mRNAs by small interfering RNAs (siRNAs), post transcriptional gene silencing (PTGs), developmentally regulated sequence-specific translational repression of mRNA by micro-RNAs (miRNAs) and targeted transcriptional gene silencing. RNAi activity limits transposon mobilization and provides an antiviral defence (Pal-Bhadra et al, 2004). A role for the RNAi machinery and small RNAs in targeting of heterochromatin complexes and epigenetic gene silencing at specific chromosomal loci has also been demonstrated (Verdel et al, 2004). Double-stranded RNA (dsRNA)-dependent post transcriptional silencing, also known as small inhibitory RNA (siRNA) or RNA interference (RNAi), is a phenomenon in which dsRNA complexes can target specific genes of homology for silencing in a short period of time. It acts as a signal to promote degradation of mRNA with sequence identity. A 20-nt siRNA is generally long enough to induce gene-specific silencing, but short enough to evade host response (Elbashir et al, 2001).

The decrease in expression of targeted gene products can be extensive with 90% silencing induced by a few molecules of siRNA.

Since the delivery of small-molecule oligonucleotides can bypass the difficulties associated with gene therapy, the use of siRNA may have advantages over traditional gene therapy. To date, efficient delivery of vector-based therapeutic genes *in vivo* remains an obstacle to successful gene therapy. It has been observed that although knockdown of the target gene by siRNA is not permanent, a single siRNA transfection can lead to a prolonged inhibition of the target protein in the parent as well as progeny cells (Tuschl, 2001). However, there is still a problem in the art in delivery of siRNA.

EP 1 249 502 A describes semiconductor nanoparticle beads having DNA covalently bonded to the surface of the beads.

WO 01/00876 A1 describes nanoparticles having oligonucleotides attached to them and uses of these in assays for detecting nucleic acids.

WO 98/04740 A describes nanoparticles having oligonucleotides attached to them and uses of these in assays for detecting nucleic acids and separating the selected nucleic acid from other nucleic acids.

EP 0 990 903 A1 describes fluorescent semiconductor nanocrystals associated to a compound and their use in biological applications.

WO 01/51665 A2 describes nanoparticles having oligonucleotides attached to them and uses of these in assays for detecting nucleic acids and separating the selected nucleic acid from other nucleic acids.

Patolsky et al., 2000, CHEMICAL COMMUNICATIONS- CHEMCOM, Vol. 6, no. 12, pages 1025-1026, describes dendritic amplification of DNA analysis by oligonucleotide-functionalized Au-nanoparticles.

Matzke et al., 2003, SCIENCE, Vol. 301, no. 5636, pages 1060-1061, reviews the extended reach of RNAi.

Miyagishi et al. 2003, ANTISENSE AND NUCLEIC ACID DRUG DEVELOPMENT, vol. 13, no. 1, pages 1-7, describes a comparison of the suppressive effects of antisense oligonucleotides and siRNAs directed against the same targets in mammalian cells.

Choudhury et al., 2004, INTERNATIONAL JOURNAL OF CANCER, Vol. 108, no. 1, pages 71-77, describes how small interfering RNA (siRNA) inhibits the expression of the Her2/Neu gene, upregulates HLA class I and induces apoptosis of Her2/Neu positive tumor cell lines.

WO 02/32404 (Consejo Superior de Investigaciones Scientificas) discloses nanoparticles formed from metal or semiconductor atoms in which ligands comprising carbohydrates are covalently linked to the core of the nanoparticles. These nanoparticles are used for modulating carbohydrate mediated interactions and are soluble and non-toxic. PCT application claiming priority from GB-A-0313259.4 (Consejo Superior de Investigaciones Scientificas and Midatech Limited) discloses magnetic nanoparticles having cores comprising passive and magnetic metal atoms, the core being covalently linked to ligands.

### Summary of the Invention

Broadly, the present invention relates to nanoparticles comprise having a core including metal and/or semiconductor atoms, the core being linked to RNA ligands. The RNA ligands are short RNA sequences designed to mimic small interfering RNA (siRNA) and micro-RNA sequences (miRNA). The nanoparticles can be used to deliver the RNA ligands and have applications in a wide range of applications, in *in vitro* systems and for therapeutic or diagnostic applications. By way of example, the nanoparticles of the present invention may be employed (1) for targeted transcriptional gene silencing, (2) for targeted mRNA degradation, (3) for imaging mRNA, (4) for inhibiting pathways by employing a plurality of RNA ligands on the same or different nanoparticles, (5) for aerosol delivery, e.g. to the lungs, (6) in combination with mRNA silencing for targeting siRNA resistant mRNA and (7) for use a tool in functional genomics.

In the art, short RNA sequences are termed "short interfering RNAs" (siRNAs) or "microRNAs" (miRNAs) depending in their origin. Both types of sequence may be used to down-regulate gene expression by binding to complimentary RNAs (nmRNA) and either triggering mRNA elimination (RNAi) or arresting mRNA translation into protein. siRNA are derived by processing of long double stranded RNAs and when found in nature are typically of exogenous origin. Micro-interfering RNAs (miRNA) are endogenously encoded small non-coding RNAs, derived by processing of short hairpins. Both siRNA and miRNA can inhibit the translation of mRNAs bearing partially complimentary target sequences without RNA cleavage and degrade mRNAs bearing fully complementary sequences. The 4 RNAi pathway also acts on the genome as discussed in Science, 301: 1060-1061, 2003).

The RNA associated with the nanoparticles may be single stranded or double stranded (duplex). Where miRNA-like sequences are used as ligands, the RNA sequences may be hairpins, that is include partially complementary regions towards their ends that can anneal to form the hairpin. The nanoparticles may optionally comprise further types of ligands, such as carbohydrates to form glyconanoparticles, and/or more than one species of siRNA. The nanoparticles and their uses are discussed in more detail below. Advantageously, the attachment of the siRNA to the nanoparticle may provide protection for the siRNA from exoribonucleases present in the blood, tissue culture media or within cells.

Accordingly, in a first aspect, the present invention provides nanoparticles for use in a method of therapy, wherein said nanoparticles comprise a core including metal and/or semiconductor atoms, and wherein the core is covalently linked to a plurality of ligands which comprise at least one RNA ligand comprising a siRNA or miRNA molecule.

The nanoparticles for use in accordance with the first aspect of the invention may be for use in a method which is an *in vivo* method of down regulating a target gene.

The nanoparticles for use in accordance with the first aspect of the invention may be for use in a method of treatment of cancer, a viral infection or macular degeneration of the eye. In some cases, the cancer is breast cancer or the virus is HIV, hepatitis or influenza.

The nanoparticles for use in accordance with the first aspect of the invention may comprise a membrane translocation signal so that they are capable of permeating through the cell membrane.

In accordance with the first aspect of the invention the nanoparticle may be covalently linked to said siRNA ligand via a linker group. In some cases, the linker group is a thiol group, an ethylene group or a peptide group.

In accordance with the first aspect of the invention the ligand may be a siRNA ligand and may comprise a 3' overhang of 2 ribonucleotides.

In accordance with the first aspect of the invention a first sense or antisense strand of a RNA molecule may be covalently linked to a nanoparticle core via its 5' and/or 3' end. In some cases a second strand of the RNA molecule which is complementary to the first strand is annealed to the first strand of the RNA molecule. In some cases the second RNA strand is covalently linked to a nanoparticle core via its 5' and/or 3' end. In some cases the first and second strands of the RNA molecule are separately linked to nanoparticle cores and subsequently annealed together.

In accordance with the first aspect of the invention the RNA ligand may be a miRNA ligand that comprises a hairpin.

In accordance with the first aspect of the invention the RNA ligand may be based on a Her2 gene sequence.

In accordance with the first aspect of the invention said RNA ligand may be single stranded or double stranded.

In accordance with the first aspect of the invention said RNA ligand may have between:
10 and 40 ribonucleotides,
17 and 30 ribonucleotides,
19 and 25 ribonucleotides, or
21 and 23 ribonucleotides.

In a second aspect, the present invention provides an *in vitro* method for down regulating a target gene, the method comprising contacting cells containing the gene with nanoparticles in accordance with the first aspect of the invention. In some cases the method produces a transient knock out of the target gene.

In some cases the method of the second aspect of the invention employs nanoparticles having at least two different RNA ligands, either conjugated to the same nanoparticles or present in a composition of at least two different types of nanoparticles, to down regulate expression in at least two genes in a pathway. In some cases the pathway is an inflammatory pathway, an anti-viral pathway, a signalling pathway in cancer, a metastasis pathway or a metabolic pathway. In some cases the RNA ligand is based on a conserved domain of a gene family to down regulate the expression of a plurality of members of the gene family.

Embodiments of the present invention will now be described by way of example with reference to the accompanying figures.

### Brief Description of the Figures

**Figure 1** shows a transmission electron micrograph of RNA-Au-Glc nanoparticles.
**Figure 2** shows the testing for the presence of RNA in the prepared nanoparticles. (a) Without UV light: 1. RNA-Au-Glc nanoparticles + EtBr; 2. Glc-Au + EtBr; 3. Glc-Au; 4. residue of washing solution+ EtBr. (b) With UV light: 1. RNA-Au-Glc nanoparticles + EtBr; 2. Glc-Au + EtBr; 3. Glc-Au; 4. residue of washing solution + EtBr.
**Figure 3a** shows a Western blot of Her-2/neu protein from equal volumes of lysates of SKBR3 cells 48 hours after transfection with a Her-2/neu siRNA. C = Control (untreated) cells; Au = Cells treated with siRNA coupled to gold nanoparticles without RNAiFectamine; S = Silencing siRNA with RNAiFectamine; NS = Non-silencing siRNA with RNAiFectamine.
**Figure 3b** shows a Western blot of Her-2/neu protein from equal volumes of OVCAR cell lysates 72 hours after transfection with a Her-2/neu siRNA. C = Control (untreated) cells; Au = Cells treated with siRNA coupled to gold nanoparticles without RNAiFectamine.
**Figure 4** shows a schematic representation of a preferred nanoparticle of the invention comprising siRNA and carbohydrate ligands.
**Figure 5** shows the effect on cell proliferation on OVCAR cells transfected with siRNA alone **(A)** or with siRNA-nanoparticles **(B)** at 0.25µg (diamonds), 0.5µg (squares), 1.0µg (triangles), 1.5µg (grey cross) and 2.0µg (black cross) siRNA-nanoparticles per 1000 cells. X axis = days; Y axis = cell number (log₁₀)
**Figure 6** shows the effect on cell proliferation on OVCAR cells transfected with siRNA-nanoparticles with and without transfection reagent. Three concentrations of nanoparticles were used:
   1: with transfection reagent (squares) and without transfection reagent (diamonds);
   2: with transfection reagent (grey crosses) and without transfection reagent (triangles);
   3: with transfection reagent (circles) and without transfection reagent (black crosses).
   X axis = days; Y axis = cell number (log₁₀)

### Detailed Description

### Examples

The Her-2/neu oncogene and its encoded product p185Her-2/neu belong to the epidermal growth factor receptor tyrosine kinases (Bargmann et al, 1986). The HER receptor family consists of four transmembrane tyrosine kinases: EGFR (also known as Her-1 or erbB-1), erbB-2 (Her-2), erbB-3 (Her-3), and erbB-4 (Her-4). Her-2/neu signalling pathways are known to play critical roles in cell growth and differentiation, malignant transformation, and resistance to chemotherapeutic agents (Yarden & Sliwkowski, 2001). Her-2/neu is over-expressed in about one third of cases of human breast or ovarian cancers, and its over-expression is associated with poor prognosis (Berchuck et al, 1990).

Numerous attempts have been made to inhibit Her-2/neu expression in cancer cells as a potential therapeutic approach. A humanized monoclonal antibody against Her-2/neu (Trastuzumab or Herceptin) has been effective in Her-2/neu-overexpressing metastatic cancer (Mendelsohn & Baselga, 2000; Baselga et al, 1996) but was found to up-regulate Her-3 expression. An antisense oligonucleotide against Her-2/neu has been shown to induce apoptosis in human breast cancer cell lines that overexpress Her-2/neu (Roh et al, 2000). Gene therapy with E1A, delivered by liposomes or by adenoviral vectors, can reduce mortality among tumour-bearing mice in a model of Her-2/neu-overexpressing ovarian cancer and can reduce the incidence of distant metastases in a model of breast cancer (Chang et al, 1996).

The down regulation of Her-2/neu expression was found to lead to decreases in PI3K, Akt, and phosphorylated Akt which resulted in decreased expression of cyclin D1, a cyclin involved in the regulation of G0/G1 cell arrest and oncogenic transformation (Sherr & Roberts, 1999). A recent study comparing the efficacy of antisense oligonucleotides and siRNA demonstrated that siRNAs are at least 10 times more efficient on a nM basis at silencing a reporter gene (Miyagishi et al, 2003). Several previous studies have demonstrated that Her-2/neu stimulates the transcription of VEGF, a potent proangiogenic factor (Kumar & Yarmand-Bagheri, 2001) the level of which was markedly decreased after silencing of Her-2/neu expression. Down regulation of Her-2/neu by retroviral siRNA increased thrombospondin-1 levels, a powerful inhibitor of angiogenesis (Izumi et al, 2002). *In vitro* data demonstrated that HER2 siRNA treatment also significantly up-regulates HLA class I surface expression in human tumours (Choudhury et al, 2004).

### a. Strategy: Silencing

Her2/Neu cDNA Target Sequence: **AAG CCT CAC AGA GAT CTT GAA**
a) Sense: **5'- G CCU CAC AGA GAU CUU GAAdTdT** - **3'**
b) Antisense: **3'** - **dTdTC GGA GUG UCU CUA GAA CUU** - **5'**
c) Sense: **5'-** G **CCU CAC AGA GAU CUU GAAdTdT-3'SS**
d) Antisense:**3**'**SS**-**dTdTC GGA GUG UCU CUA GAA CUU** - **5'**

### b. Annealing

| | |
|---|---|
| Silencing: | 5′ (a) 3′ 3′ (b) 5′ |
| | 5′ (a) 3′ 3′SS (d) 5′ |
| | 5′ (c) 3′SS 3′ (b) 5′ |
| | 5′ (c) 3′SS 3SS′ (d) 5′ |

### c. Possible GNP combinations

| **siRNA** | **Anneal** | **GNP** | | |
|---|---|---|---|---|
| | | **Glc**- | **Glcβ4GlcNAc** | **Glcβ4GlcNH₂** |
| **Silencing** | a-b | | | |
| | a-d | | | |
| | c-b | X | | |
| | c-d | | | |

| | | | | |
|---|---|---|---|---|
| X = used in this study. | | | | |

### d. Methods

### 1. Cell Lines

SK-BR-3 Human Mammary adenocarcinoma from ATCC (Cat.# HTB-30). OVCAR-3 Human ascites adenocarcinoma from NCI-Frederick Cancer DCTD Tumor/cell line repository (vial 0502296).

### 2. siRNA Stock Solutions

The Her-2/neu DNA target Sequence chosen was AAGCCTCACA GAGATCTTGAA.

The sense siRNA had a sequence r(GCCUCACAGAGAUCUUGAA) d(TT)3ThSS. (MW of K-salt 7416.25) and the antisense sequence r(UUCAAGAUCUCUGUGAGGC) d(TT)3ThSS (MW of K-salt 7409.57) were obtained from Qiagen.

The control (non-silencing) siRNA duplex sequences from Qiagen (Cat# 1022076) where sense r(UUC UCC GAA CGU GUC ACG U)d(TT) and antisense r(ACG UGA CAC GUU CGG AGA A)d(TT) and the MW of the annealed K-salt was 14839.5.

Dissolve contents (296.65 µg) of one sense siRNA tube in 1 ml sterile buffer (100 mM potassium acetate, 30 mM Hepes-KOH, 2 mM magnesium acetate pH 7.4) to make a 40 µM stock. Each µl will contain 0.297 µg siRNA.

The contents (296.38 µg) of one antisense siRNA tube were dissolved in 1 ml sterile buffer (100 mM potassium acetate, 30 mM Hepes-KOH, 2 mM magnesium acetate pH 7.4) to make a 40 µM stock. Each µl contained 0.296 µg siRNA.

To anneal, 30 µl of each RNA oligo solution was combined with 15 µl of 5X annealing buffer. The final buffer concentration was 50 mM Tris, pH 7.5 - 8.0, 100 mM NaCl in DEPC-treated water. The final volume was 75 µl and the final concentration of siRNA duplex was 16 µM.

The solution was incubated for 1 minute in a water bath at 90-95°C, and allowed to cool to room temperature (i.e. below 30°C). The tube was centrifuged briefly to collect all liquid at the bottom of the tube. Slow cooling to room temperature took 45-60 minutes. The resultant solution was stored at -20°C until ready to use and was resistant to repeated freezing and thawing.

### 3. siRNA Nanogold Stock Solution

### General Methods

HAuCl₄ (99.999%) and NaBH₄ were purchased from Aldrich Chemical Company. 2-thioethyl-β-D-glucopyranoside was synthesized in our laboratory using standard procedures. For all experiments and solutions, Nanopure water (18.1 mΩ) treated with DEPC (diethylpirocarbonate) was used. All eppendorfs, spatulas and vials were RNase free. Annealed double-stranded siRNA was purchased from Qiagen-Xeragon Inc. The specifications were:
DNA target sequence AAGCCTCACAGAGATCTTGAA.
Sense siRNA r (GCCUCACAGAGACUUGAA) d (TT) 3'-Thiol-(SS)-C3-linker on 3'
   (MW of K-salt 7416.25)
Antisense r(UUCAAGAUCUCUGUGAGGC)d(TT)
   (MW of K-salt 7409.57)

### e. Preparation of RNA-Au-Glc nanoparticles

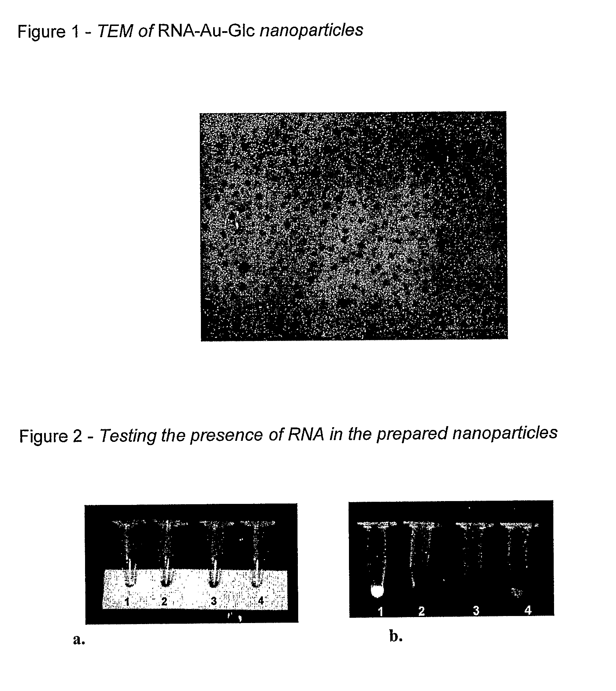

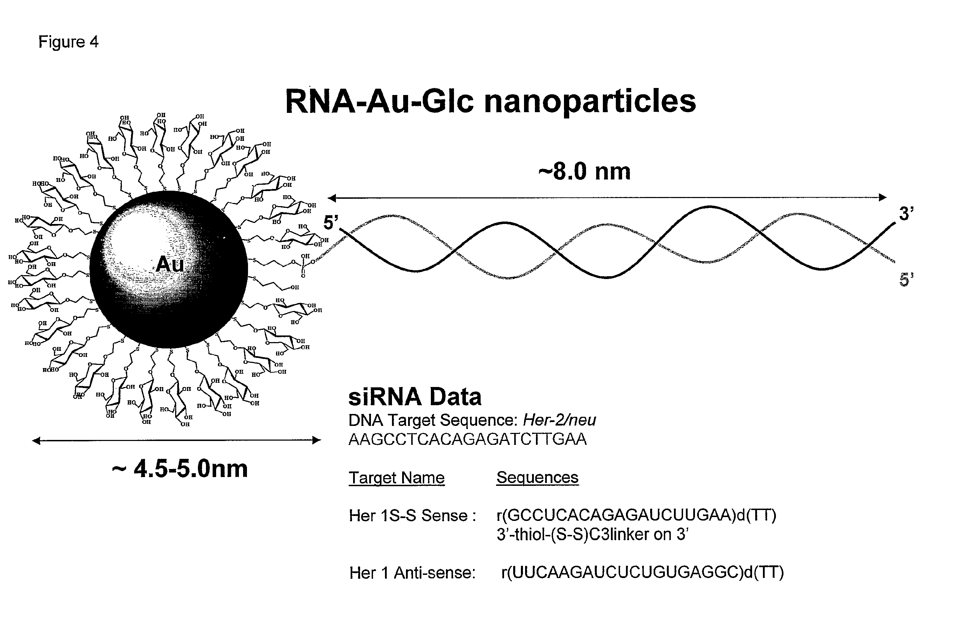

To a solution of 2-thioethyl-β-D-glucopyranoside (0.9 mg, 3.75 µmol) and siRNA (0.148 mg, 0.01 µmol) in TRIS buffer 100 mM, pH 7.7 (250 µL), an aqueous HAuCl₄ solution (22 µL, 0.025M) was added. Then, IN aqueous solution of NaBH₄ (30 µL) was added in several portions with rapid shaking. The brown suspension formed was shaken for an additional 1h at 4°C. The suspension was purified by centrifugal filtering (AMICON MW 10000, 30min, 4°C, 14000 rpm). The process was repeated twice, washing with 125 µL of TRIS buffer. The residue in the AMICON filter was dissolved in 250 µL of TRIS buffer and lyophilised to afford 4 mg of RNA-Au-Glc nanoparticles (resuspension of the solid in 1mL of water should give a 6±1µM solution of RNA in 20 mM TRIS buffer). The filtrate was desalted using AMICON (MW 3000, 4°C, 14000 rpm) and lyophilised. The weight of the residue was < 50 µg. The transmission electron micrograph (TEM) shown in Figure 1 shows that the average size of the particles was 2.8 nm, with an average of 807 gold atoms/particle, siRNA and 100 molecules of the glucose derivative as represented in Figure 4 and has an approximate MW >160,000.

### f. Checking the presence of RNA in nanoparticles

RNA-Au-Glc nanoparticles, Glc-Au nanoparticles and the residue of washing the RNA-Au-Glc nanoparticles which presumably containing RNA oligonucleotide and glucose derivative were each dissolved in 30µL of water. Aliquots of these solutions (1µL) were mixed with aqueous solution of ethidium bromide (EtBr) (1 µL, 0.1% v/v). Fluorescence was observed under a UV lamp (see Figure 2) *demonstrating* that the so-prepared nanoparticles have incorporated siRNA (figure 2b, tube 1), while the nanoparticles containing only glucose do not show any fluorescence (figure 2b, tube 2).

4 mg of the siRNA/nanogold complex generated from 148 µg siRNA after were dissolved in 1 ml water to obtain a 6±1 µM stock solution in 20 mM tris. Each µl of solution contained the equivalent of 0.078 µg siRNA.

### Cell Plating

1. 24 h before transfection, 6 x 10⁴ cells were pipetted into a 24-well plate and the volume made up to 0.5 ml with appropriate culture medium.
2. The cells were allowed to reach 50-80 % confluency, taking approximately 24 h.
3. The culture medium was removed and replaced with 300 µl fresh medium/well.

### Transfecting cells with siRNA Complex

1. 3.3 µl (or 12.8 µl of the nanogold complex) of the appropriate duplex siRNA stock was dispensed into a 24-well plate corresponding to that with cells.
2. To each well 96.7 µl (or 87.2 µl for the nanogold complex) of the appropriate culture medium was added and mixed well by pipetting up and down 5 times.
3. To each well (other than the nanogold complex wells) 6 µl of RNAiFect was added and mixed well by pipetting up and down 5 times.
4. The solutions were incubated at room temperature for 10-15 minutes to allow complex formation.
5. The cells in 300 µl culture medium were overlayed with the 100 µl of the appropriate transfection complex.
6. The plate gently rocked to mix avoiding swirling.
7. The plate was incubated at 37°C in a CO₂ incubator for 48-72 h.
8. The medium was removed and the cells washed three times with ice cold PBS.
9. The cells were lysed and the protein content of the lysates determined.
10. The proteins were separated by SDS-PAGE followed by Western blotting using a Her2/ErbB2 polyclonal rabbit antibody from Cell Signalling Technology (Cat.# 2242).
11. The blots were treated with an anti-rabbit IgG-HRP conjugate followed by ECL development.

### g. Results

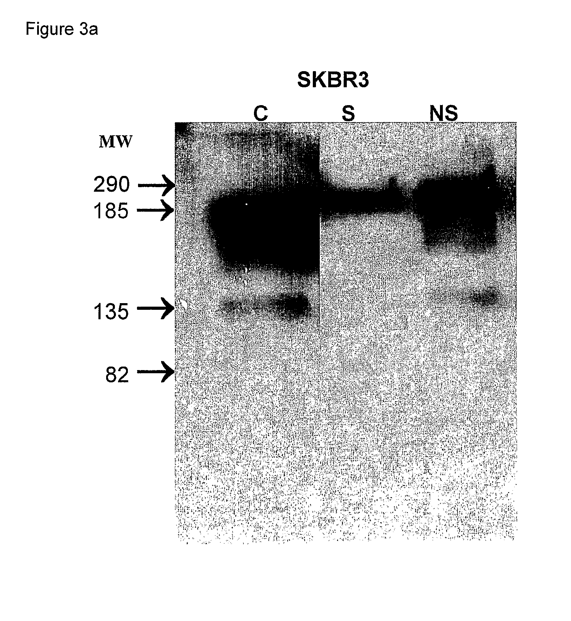

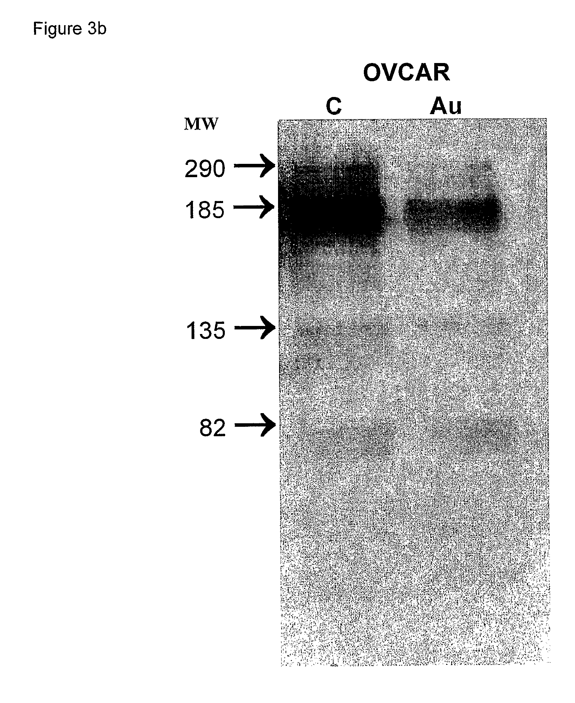

Preliminary observations using 1µg siRNA/well are shown in Figures 3a and 3b. siRNA-gold nanoparticles were added to cells without RNAiFectamine. The SKBR3 cells were slower to reach 80% confluency than the OVCAR cells. The SKBR3 results are from lysates 48h after transfection while the OVCAR results were from lysates 72h after transfection. A schematic of the nanoparticles are shown in Figure 4.

### Non-toxicity of siRNA-Au-Glc nanoparticles to cells

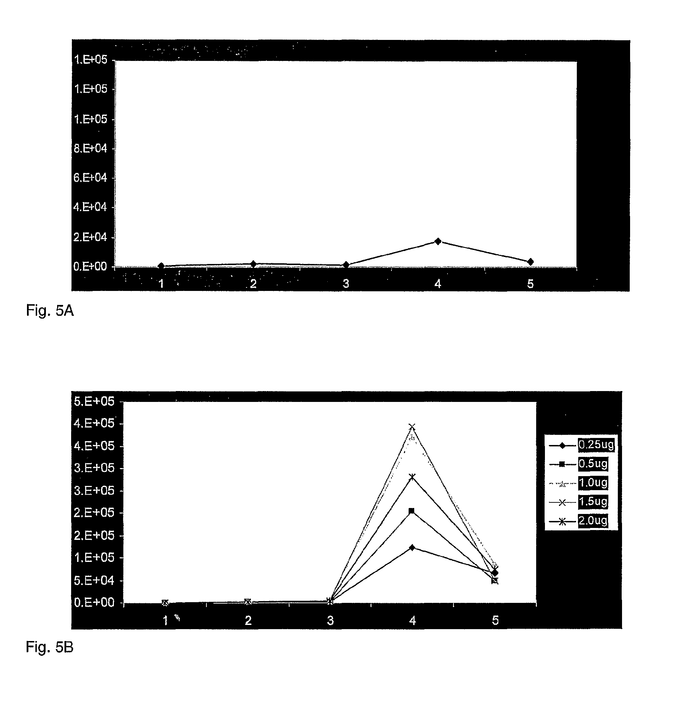

Cells were transfected with siRNA alone and with siRNA conjugated to gold glyconanoparticles. Figure 5 shows that the nanoparticle-conjugated siRNA was effective and had no toxic effects. A dose-dependent effect on cell number was seen indicating that the siRNA nanoparticles increased cell proliferation.

### Entry of siRNA-Au-Glc nanoparticles into cells

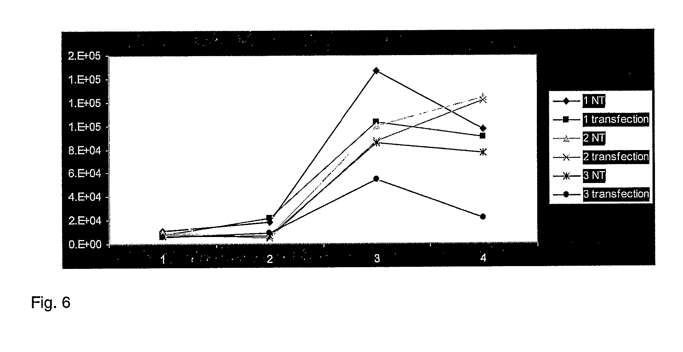

OVCAR cells were transfected with siRNA-nanoparticles with and without the transfection reagent usually required for transfection of cells with siRNA. Figure 6 shows that the transfection reagent was not required for entry of siRNA-nanoparticles into the cells. The results show that the siRNA nanoparticles were effectively delivered into the cells even in the absence of transfection reagent; indeed, delivery appeared tobe more efficient without transfection reagent. The dose-dependency of the effect on cell number indicates a genuine response to the siRNA-nanoparticles.

### References

Pal-Bhadra et al. RNAi-Mediated targeting of Heterochromatin by the RITS complex. Science (2004) vol. 303, 669.
Verdel et al. Heterochromatic silencing and HP1 localization in Drosphila are dependent on the RNAi Machinery. Science (2004) vol. 303, 672.
Elbashir et al. Duplexes of 21-nucleotide RNAs mediate RNA interference in cultured mammalian cells. Nature 2001;411:494-8.
Tuschl. RNA interference and small interfering RNAs. Chem Biochem 2001;2:239-45.
Bargmann et al. The neu oncogene encodes an epidermal growth factor receptor-related protein. (1986) Nature 319, 226-230.
Yarden & Sliwkowski. Untangling the ErbB signalling network. (2001) Nat Rev Mol Cell Biol 2, 127-137.
Berchuck et al. Overexpression of HER-2/neu is associated with poor survival in advanced epithelial ovarian cancer. (1990) Cancer Res 50, 4087-4091.
Mendelsohn & Baselga. The EGF receptor family as targets for cancer therapy. (2000) Oncogene 19, 6550-6565.
Baselga et al. Phase II study of weekly intravenous recombinant humanized anti-p185HER2 monoclonal antibody in patients with HER2/neu over expressing metastatic breast cancer. J Clin Oncol 1996;14:737-44.
Roh et al. Down regulation of HER2/neu expression induces apoptosis in human cancer cells that over-express HER2/neu. (2000) Cancer Res 60, 560-565.
Chang et al. Inhibition of intratracheal lung cancer development by systemic delivery of E1A. (1996) Oncogene 13, 1405-1412.
Sherr & Roberts. CDK inhibitors: positive and negative regulators of G1-phase progression. (1999) Genes Dev 13, 1501-1512.
Miyagishi et al. Comparison of the suppressive effects of antisense oligonucleotides and siRNAs directed against the same targets in mammalian cells. Antisense Nucleic Acid Drug Dev 2003;13:1-7.
Kumar & Yarmand-Bagheri. The role of HER2 in angiogenesis. (2001) Semin Oncol 28, 27-32.
Izumi et al. Tumour biology: herceptin acts as an antiangiogenic cocktail. (2002) Nature 416, 279-280.
Choudhury et al. Small interfering RNA (siRNA) inhibits the expression of the Her2/Neu gene, upregulates HLA Class I and induces apoptosis of Her2/Neu positive tumour cell lines. Int J Cancer 108, 71-77, 2004.
Overbaugh, HTLV sweet-talks its way into cells, Nat. Med (2004) vol. 10, 20.
Check. RNA to the rescue. Nature (2003)vol 425, 10 - 12.
Zamore et al. siRNAs knock down hepatitis. Nature (2003) vol 9, 266-267.
Song et al. RNA interference targeting Fas protects mice from fulminant hepatitis. Nat. Med. (2003)vol. 9, 347-351.
Matzke & Matzke. RNAi Extends its Reach. Science (2003) Vol 301, 1060 - 1061.
WO 02/32404
PCT application claiming priority from GB-A-0313259.4.

## Claims

1. Nanoparticles for use in a method of therapy, wherein said nanoparticles comprise a core including metal and/or semiconductor atoms, and wherein the core is covalently linked to a plurality of ligands which comprise at least one RNA ligand comprising a siRNA or miRNA molecule.

2. Nanoparticles for use in accordance with claim 1, wherein said method is an *in vivo* method of down regulating a target gene.

3. Nanoparticles for use in a method of treatment of cancer, a viral infection or macular degeneration of the eye, wherein said nanoparticles are as defined in claim 1.

4. Nanoparticles for use in accordance with claim 3, wherein the cancer is breast cancer or the virus is HIV, hepatitis or influenza.

5. Nanoparticles for use in accordance with any one of claims 1 to 4, wherein the nanoparticles comprise a membrane translocation signal so that they are capable of permeating through the cell membrane.

6. Nanoparticles for use in accordance with any one of the preceding claims, wherein the nanoparticle is covalently linked to said siRNA ligand via a linker group.

7. Nanoparticles for use in accordance with claim 6, wherein the linker group is a thiol group, an ethylene group or a peptide group.

8. Nanoparticles for use in accordance with any one of the preceding claims, wherein the ligand is a siRNA ligand and comprises a 3' overhang of 2 ribonucleotides.

9. Nanoparticles for use in accordance with any one of the preceding claims, wherein a first sense or antisense strand of a RNA molecule is covalently linked to a nanoparticle core via its 5' and/or 3' end.

10. Nanoparticles for use in accordance with claim 9, wherein a second strand of the RNA molecule which is complementary to the first strand is annealed to the first strand of the RNA molecule.

11. Nanoparticles for use in accordance with claim 10, wherein the second RNA strand is covalently linked to a nanoparticle core via its 5' and/or 3' end.

12. Nanoparticles for use in accordance with claim 10 or claim 11, wherein the first and second strands of the RNA molecule are separately linked to nanoparticle cores and subsequently annealed together.

13. Nanoparticles for use in accordance with any one of the preceding claims, wherein the RNA ligand is a miRNA ligand and comprises a hairpin.

14. Nanoparticles for use in accordance with any one of the preceding claims, wherein the RNA ligand is based on a Her2 gene sequence.

15. Nanoparticles for use in accordance with any one of the preceding claims, wherein said RNA ligand is single stranded or double stranded.

16. Nanoparicles for use in accordance with any one of the preceding claims, wherein said RNA ligand has between:
10 and 40 ribonucleotides,
17 and 30 ribonucleotides,
19 and 25 ribonucleotides, or
21 and 23 ribonucleotides.

17. An *in vitro* method for down regulating a target gene, the method comprising contacting cells containing the gene with nanoparticles as defined in any one of the preceding claims.

18. The method of claim 17, wherein the method produces a transient knock out of the target gene.

19. The method of claim 17 or claim 18, wherein the method employs nanoparticles having at least two different RNA ligands, either conjugated to the same nanoparticles or present in a composition of at least two different types of nanoparticles, to down regulate expression in at least two genes in a pathway.

20. The method of claim 19, wherein the pathway is an inflammatory pathway, an anti-viral pathway, a signalling pathway in cancer, a metastasis pathway or a metabolic pathway.

21. The method of any one of claims 17 to 20, wherein the RNA ligand is based on a conserved domain of a gene family to down regulate the expression of a plurality of members of the gene family.

## Patentansprüche

1. Nanopartikel zur Verwendung in einem Therapieverfahren, worin die Nanopartikel einen Kern umfassen, der Metall- und/oder Halbleiteratome umfasst, und worin der Kern kovalent an eine Vielzahl von Liganden gebunden ist, die zumindest einen RNA-Liganden umfassen, der ein siRNA- oder miRNA-Molekül umfasst.

2. Nanopartikel zur Verwendung nach Anspruch 1, worin das Verfahren ein *Invivo*-Verfahren zur Herabregulierung eines Zielgens ist.

3. Nanopartikel zur Verwendung in einem Verfahren zur Behandlung von Krebs, einer Vireninfektion oder von Makuladegeneration am Auge, worin die Nanopartikel so sind wie in Anspruch 1 definiert.

4. Nanopartikel zur Verwendung nach Anspruch 3, worin der Krebs Brustkrebs ist oder das Virus HIV, Hepatitis oder Influenza ist.

5. Nanopartikel zur Verwendung nach einem der Ansprüche 1 bis 4, worin die Nanopartikel ein Membrantranslozierungssignal umfassen, sodass sie in der Lage sind, die Zellmembran zu durchdringen.

6. Nanopartikel zur Verwendung nach einem der vorangegangenen Ansprüche, worin das Nanopartikel kovalent über eine Linkergruppe an den siRNA-Liganden gebunden ist.

7. Nanopartikel zur Verwendung nach Anspruch 6, worin die Linkergruppe eine Thiolgruppe, eine Ethylengruppe oder eine Peptidgruppe ist.

8. Nanopartikel zur Verwendung nach einem der vorangegangenen Ansprüche, worin der Ligand ein siRNA-Ligand ist und einen 3'-Überhang aus 2 Ribonucleotiden umfasst.

9. Nanopartikel zur Verwendung nach einem der vorangegangenen Ansprüche, worin ein erster Sense- oder Antisense-Strang eines RNA-Moleküls über dessen 5'-und/oder 3'-Ende kovalent an einen Nanopartikelkern gebunden ist.

10. Nanopartikel zur Verwendung nach Anspruch 9, worin ein zweiter Strang des RNA-Moleküls, der zu dem ersten Strang komplementär ist, an den ersten Strang des RNA-Moleküls anelliert ist.

11. Nanopartikel zur Verwendung nach Anspruch 10, worin der zweite RNA-Strang über dessen 5'- und/oder 3'-Ende kovalent an einen Nanopartikelkern gebunden ist.

12. Nanopartikel zur Verwendung nach Anspruch 10 oder Anspruch 11, worin der erste und der zweite Strang des RNA-Moleküls getrennt voneinander an Nanopartikelkerne gebunden und in weiterer Folge aneinander anelliert sind.

13. Nanopartikel zur Verwendung nach einem der vorangegangenen Ansprüche, worin der RNA-Ligand ein miRNA-Ligand ist und eine Haarnadel umfasst.

14. Nanopartikel zur Verwendung nach einem der vorangegangenen Ansprüche, worin der RNA-Ligand auf einer Her2-Gensequenz basiert.

15. Nanopartikel zur Verwendung nach einem der vorangegangenen Ansprüche, worin der RNA-Ligand einzelsträngig oder doppelsträngig ist.

16. Nanopartikel zur Verwendung nach einem der vorangegangenen Ansprüche, worin der RNA-Ligand:
zwischen 10 und 40 Ribonucleotide,
zwischen 17 und 30 Ribonucleotide,
zwischen 17 und 30 Ribonucleotide oder
zwischen 21 und 23 Ribonucleotide aufweist.

17. *In-vitro*-Verfahren zur Herabregulierung eines Zielgens, wobei das Verfahren das Kontaktieren von das Gen enthaltenden Zellen mit in einem der vorangegangenen Ansprüche definierten Nanopartikeln umfasst.

18. Verfahren nach Anspruch 17, worin das Verfahren ein vorübergehendes Knockout des Zielgens herbeiführt.

19. Verfahren nach Anspruch 17 oder Anspruch 18, worin im Verfahren Nanopartikel mit zumindest zwei verschiedenen RNA-Liganden, entweder an dieselben Nanopartikel konjugiert oder in einer Zusammensetzung mit zumindest zwei verschiedenen Arten von Nanopartikeln, eingesetzt werden, um die Expression zumindest zweier Gene auf einem Weg herabzuregulieren.

20. Verfahren nach Anspruch 19, worin der Weg ein Entzündungsweg, ein antiviraler Weg, ein Signalgebungsweg, ein Metastasenweg oder ein Stoffwechselweg ist.

21. Verfahren nach einem der Ansprüche 17 bis 20, worin der RNA-Ligand auf einer konservierten Domäne einer Genfamilie basiert, um die Expression einer Vielzahl von Angehörigen der Genfamilie herabzuregulieren.

## Revendications

1. Nanoparticules destinées à être utilisées dans un procédé de thérapie, où lesdites nanoparticules comprennent un noyau comprenant des atomes métalliques ou semiconducteurs, et où le noyau est lié de façon covalente à une pluralité de ligands qui comprennent au moins un ligand d'ARN comprenant une molécule d'ARNsi ou d'ARNmi.

2. Nanoparticules destinées à être utilisées selon la revendication 1, où ledit procédé est un procédé *in vivo* de régulation négative d'un gène cible.

3. Nanoparticules destinées à être utilisées dans un procédé de traitement du cancer, d'une infection virale ou d'une dégénérescence maculaire de l'oeil, où lesdites nanoparticules sont telles que définies dans la revendication 1.

4. Nanoparticules destinées à être utilisées selon la revendication 3, où le cancer est le cancer du sein ou le virus est le VIH, l'hépatite ou la grippe.

5. Nanoparticules destinées à être utilisées selon l'une quelconque des revendications 1 à 4, où les nanoparticules comprennent un signal de translocation membranaire de sorte qu'elles soient capables de traverser la membrane cellulaire.

6. Nanoparticules destinées à être utilisées selon l'une quelconque des revendications précédentes, où la nanoparticule est liée de façon covalente audit ligand d'ARNsi via un groupe de liaison.

7. Nanoparticules destinées à être utilisées selon la revendication 6, où le groupe de liaison est un groupe thiol, un groupe éthylène ou un groupe peptidique.

8. Nanoparticules destinées à être utilisées selon l'une quelconque des revendications précédentes, où le ligand est un ligand d'ARNsi et comprend un porte-à-faux de 2 ribonucléotides en 3'.

9. Nanoparticules destinées à être utilisées selon l'une quelconque des revendications précédentes, où un premier brin sens ou antisens d'une molécule d'ARN est lié de façon covalente à un noyau de nanoparticule via son extrémité 5' et/ou 3'.

10. Nanoparticules destinées à être utilisées selon la revendication 9, où un second brin de la molécule d'ARN qui est complémentaire au premier brin est annelé au premier brin de la molécule d'ARN.

11. Nanoparticules destinées à être utilisées selon la revendication 10, où le second brin d'ARN est lié de façon covalente à un noyau de nanoparticule via son extrémité 5' et/ou 3'.

12. Nanoparticules destinées à être utilisées selon la revendication 10 ou la revendication 11, où les premier et second brins de la molécule d'ARN sont séparément liés à des noyaux de nanoparticules et sont ensuite annelés ensemble.

13. Nanoparticules destinées à être utilisées selon l'une quelconque des revendications précédentes, où le ligand d'ARN est un ligand d'ARNmi et comprend une épingle à cheveux.

14. Nanoparticules destinées à être utilisées selon l'une quelconque des revendications précédentes, où le ligand d'ARN est basé sur une séquence du gène Her2.

15. Nanoparticules destinées à être utilisées selon l'une quelconque des revendications précédentes, où ledit ligand d'ARN est simple brin ou double brin.

16. Nanoparticules destinées à être utilisées selon l'une quelconque des revendications précédentes, où ledit ligand d'ARN possède entre :
10 et 40 ribonucléotides,
17 et 30 ribonucléotides,
19 et 25 ribonucléotides, ou
21 et 23 ribonucléotides.

17. Procédé *in vitro* pour négativement réguler un gène cible, le procédé comprenant la mise en contact de cellules contenant le gène avec des nanoparticules telles que définies dans l'une quelconque des revendications précédentes.

18. Procédé selon la revendication 17, où le procédé produit une invalidation (knock-out) transitoire du gène cible.

19. Procédé selon la revendication 17 ou la revendication 18, où le procédé emploie des nanoparticules comportant au moins deux ligands d'ARN différents, qui sont soit conjugués aux mêmes nanoparticules ou sont présents dans une composition d'au moins deux types de nanoparticules différents, afin de négativement réguler l'expression d'au moins deux gènes dans une voie.

20. Procédé selon la revendication 19, dans lequel la voie est une voie inflammatoire, une voie antivirale, une voie de signalisation dans le cancer, une voie métastatique ou une voie métabolique.

21. Procédé selon l'une quelconque des revendications 17 à 20, dans lequel le ligand d'ARN est basé sur un domaine conservé d'une famille de gènes afin de négativement réguler l'expression d'une pluralité de membres de la famille de gènes.
